(19) **Europäisches Patentamt**

**European Patent Office**

**Office européen des brevets**

(11) **EP 0 573 765 B1**

(12) **EUROPÄISCHE PATENTSCHRIFT**

(45) Veröffentlichungstag und Bekanntmachung des Hinweises auf die Patenterteilung:
**26.11.2003 Patentblatt 2003/48**

(51) Int Cl.⁷: **A61B 5/113**, G01L 1/24

(21) Anmeldenummer: **93106744.1**

(22) Anmeldetag: **26.04.1993**

(54) **Verfahren und Vorrichtung zur Überwachung der Änderung des Bewegungszustandes von Gegenständen oder Teilen des menschlichen Körpers**

Method and device for monitoring the change of movement of items or parts of the human body

Méthode et dispositif de surveillance de la variation d'état de mouvement des articles ou des parties du corps humain

(84) Benannte Vertragsstaaten:
**AT DE ES FR GB IT NL SE**

(30) Priorität: **24.04.1992 US 874297**

(43) Veröffentlichungstag der Anmeldung:
**15.12.1993 Patentblatt 1993/50**

(73) Patentinhaber: **Buschmann, Johannes**
**81669 München (DE)**

(72) Erfinder: **Buschmann, Johannes**
**81669 München (DE)**

(56) Entgegenhaltungen:
**EP-A- 0 198 545**    **WO-A-88/02237**
**WO-A-88/08687**    **DE-A- 3 935 083**
**GB-A- 1 596 298**    **US-A- 4 734 577**
**US-A- 5 134 281**

**Beschreibung**

Die Erfindung betrifft ein Verfahren und eine Vorrichtung zur Überwachung der Änderung des Bewegungszustandes von Gegenständen oder Teilen des menschlichen Körpers nach Anspruch 1 bzw. Anspruch 12.

Derartige Verfahren und Vorrichtungen werden auf unterschiedlichsten Gebieten der Technik und der Medizin benötigt. Beispiele hierfür sind Bewegungsmelder oder Alarmanlagen, die beispielsweise zur Sicherung von Kunstgegenständen wie Bildern oder Plastiken, verwendet werden. Die Änderung des Bewegungszustandes stellt in diesem Falle das Anheben eines Gegenstandes oder das Abhängen eines Bildes dar, also der Wechsel vom Ruhezustand in einen zumindest zeitweise bewegten Zustand.

In der Maschinentechnik können derartige Verfahren und Vorrichtungen beispielsweise dazu verwendet werden, unerwünscht hohe Schwingungszustände einer Maschine, beispielsweise beim Auftreten von Unwuchten, zu erfassen und durch einen Alarm anzuzeigen.

In der Medizintechnik werden solche Verfahren und Vorrichtungen zur Überwachung unterschiedlicher Zustände des menschlichen Körpers verwendet, die sich in der Änderung des Bewegungszustandes eines Körperteiles äußern. Ein Beispiel hierfür stellt das Phänomen des plötzlichen Kindstodes, in der medizinischen Literatur mit "SIDS" abgekürzt (= Sudden Infant Death Syndrom) dar. Der Grund für diesen plötzlichen Kindstod ist ein zentraler Atemstillstand unbekannter Ursache. Dieses Beispiel aus dem Bereich der Medizin ist insofern besonders wichtig, als SIDS alle Kinder in ihrem ersten Lebensjahr bedroht. Der plötzliche Kindstod ist für fast die Hälfte aller Todesursachen zwischen dem 2. und 12. Lebensmonat verantwortlich.

Die Häufigkeit von SIDS variiert von Land zu Land stark: von 0,1 auf 1000 Kinder in Hongkong bis 5 auf 1000 in Neuseeland (deutsches Ärzteblatt 88, Heft 48, 28.11.1991) Da die Ursachen für die unterschiedliche Häufigkeit des plötzlichen Kindstodes unbekannt sind, und sich die Risiken beispielsweise durch Schlafen in Bauchlage, Frühgeburtlichkeit, ungenügende Schwangerschaftsbetreuung, Rauchen während der Schwangerschaft und erhöhte Raumtemperatur im Kinderzimmer erhöhen, ist letztendlich das einzige Mittel den plötzlichen Kindstod zu vermeiden, die unterbrechungsfreie Überwachung des Kindes in der gesamten gefährdeten Zeit zwischen dem 2. und 12. Lebensmonat.

Als Überwachungsmöglichkeiten sind bisher folgende Verfahren bekannt geworden:

- Sensoren, die respiratorische Bewegungen aufnehmen;
- Geräte zur Messung der Thorax-Impedanz;
- Sensoren für die Herztätigkeit; wie z.B. EKG-Geräte;
- Induktions-Plethysmographen;
- Pulsoximeter.

Sämtliche Neuerungen, Weiterentwicklungen und bekannten Verfahren bzw. Geräte zu deren Verwirklichung betreffen im wesentlichen die Unterdrückung von Artefakten (Fehlalarmen).

Die Benutzung von Thorax-Elektroden beinhaltet das Problem der Kontaktunsicherheit mit der Konsequenz von Fehlalarmen die sowohl Eltern als auch Pflegepersonal so lange in Angst und Schrecken versetzen, wie sie auf diese Alarme reagieren. Wenn sich dieser Personenkreis nach einer Weile von häufigen Fehlalarmen nicht mehr erschrecken läßt, stellt dies letztlich eine Verringerung der Alarmwirkung dar.

Fehlalarme sind überhaupt bei allen bisher bekannten Methoden des Überwachens des plötzlichen Kindstodes das größte Problem.

Falsch positive Alarme (das Gerät gibt Alarm, obwohl das Kind normal atmet), werden durch Sensoren hervorgerufen, die einen Wackelkontakt bzw. einen unterbrochenen Kontakt aufweisen. Falsch negative Alarme (das Gerät gibt keinen Alarm, obwohl das Kind einen Atemstillstand hat), passieren, wenn die Alarme abgeschaltet wurden (infolge häufiger falsch positiver Alarme), oder wenn die Sensivität des Gerätes unzureichend ist. Ein anderes Problem ist die Störanfälligkeit beim Befestigen der Sensoren wie z.B. Aufkleben bzw. andere Methoden der Fixierung von Sensoren am kindlichen Körper und die hohen Kosten von Sensoren und der Geräte.

Diese zuvor genannten Probleme treten jedoch nicht nur bei Anwendungen im Bereich der Medizin, sondern auch den herkömmlichen Überwachungsverfahren und -vorrichtungen auf, die in den eingangs genannten Gebieten der Technik für unterschiedliche Zwecke Anwendung finden.

Weiterhin ist es aus CA-A-2130429 bereits bekannt, eine Überwachung der Änderung von Bewegungszuständen, z. B. eines menschlichen Körpers durchzuführen, wobei eine optische Faser, die in gutem mechanischem Kontakt mit dem zu bewachenden Gegenstand oder Körper steht, von Licht durchflutet wird und die Veränderungen der Intensität des transmittierten Lichts, die aufgrund der Bewegung der optischen Faser über eine mechanische Kopplung mit einem Körperteil entsteht, überwacht und bei Über- oder Unterschreitung eines. Schwellenwertes ein Alarm ausgelöst wird.

Zum einen ist unklar, ob dort eine Amplitudenüberwachung des in ein elektrisches Signal umgewandelten transmittierten Lichts durchgeführt wird. Zum anderen erfolgt kein Aufmodulieren einer höheren Frequenz auf die Lichtquelle mit späterer Demodulation, die die Empfindlichkeit des Sensors deutlich steigert.

Es ist daher Aufgabe der vorliegenden Erfindung, ein Verfahren und eine Vorrichtung zur Überwachung des Änderungszustandes von Gegenständen oder Teilen des menschlichen Körpers zu schaffen, die die zuvor erläuterten Probleme herkömmlicher Verfahren und Vorrichtungen betreffend das Auslösen von Fehlalarmen, ihren komplizierten Aufbau und ihre Betriebsunsicherheit beseitigen.

Die Lösung dieser Aufgabe erfolgt durch die Merkmale des Anspruchs 1 bzw. des Anspruchs 12.

Gemäß den Prinzipien der Erfindung wird der zur Detektierung verwendete Lichtleiter in unmittelbaren Kontakt zum zu überwachenden Gegenstand bzw. zu der überwachenden Person gebracht, so daß sich alle Bewegungen bzw. Änderungen der Bewegung auf den Lichtleiter übertragen. Dies ruft eine Änderung der Geometrie des Lichtleiters bzw. eine Änderung seiner Lage im Raum hervor. Wird der Lichtleiter mit Licht durchstrahlt verursacht jede Änderung der Geometrie (Lage) eine Änderung der Transmission (Rauschen, Noise). Innerhalb bestimmter Grenzen gilt, daß das beschriebene Transmissionsrauschen desto stärker ist, je größer die Bewegung des Lichtleiters ist. Es ist daher notwendig eine (quantitative) Schwelle einzuführen, mit deren Hilfe man zwischen dem Bereich "bewegte Fasern" und "unbewegte Fasern" unterscheiden kann. Diese Schwelle erhält man durch Einführung eines Amplitudenkomparators: Es wird eine Spannung vorgegeben bzw. eingestellt deren Überschreitung als Bewegung verstanden wird deren Unterschreiten (noch) als Ruhezustand interpretiert wird.

Zu den besonderen Vorteilen des erf indungsgemäßen Verfahrens und der erfindungsgemäßen Vorrichtung zählt, daß auf zuverlässige Art und Weise erfaßt werden kann, ob eine Bewegung oder eine Änderung des Bewegungszustandes vorliegt oder nicht. Im Falle des Überwachens von Kleinkindern wird dies beispielsweise bedeuten, daß auf zuverlässige Art und Weise ein nach den Prinzipien des erfindungsgemäßen Verfahrens funktionierendes Gerät detektiert, ob und wann das Kind aufhört zu atmen.

Ferner ist es mit dem erfindungsgemäßen Verfahren und der erfindungsgemäßen Vorrichtung möglich, Bewegungsmuster zu detektieren, auszuwerten und aufzuzeichnen und daraus eventuell eine kontinuierliche Statistik der Bewegungsmuster zu erstellen und einen Alarm auszugeben, wenn der momentane erfaßte Zustand außerhalb von durch das Bewegungsmuster bestimmte Toleranzgrenzen liegt. Im Falle der medizintechnischen Anwendung kann ein solches Bewegungsmuster ein Atmungsmuster sein, wobei dann ein Alarm ausgegeben wird, wenn die Zeit seit der letzten Atmung eine definierte, eingestellte Zeit überschreitet (Atemstillstand).

Die Unteransprüche haben vorteilhafte Weiterbildungen der Erfindung zum Inhalt.

Vorteilhafterweise wird die optische Faser bzw. der Lichtleiter in engen mechanischen Kontakt mit dem zu überwachenden Objekt bzw. der zu beobachtenden Person gebracht. Hierzu ist es möglich, die Lichtleiter in Befestigungsvorrichtungen unterzubringen, die an dem jeweiligen Objekt bzw. an der Person befestigt werden können.

Zur Beschickung der Lichtleiter mit Licht ist es möglich, Lichtquellen geeigneter Art, z.B. LEDs oder Halbleiterlaser (Laserdioden), Leuchtdioden zu verwenden. Diese senden Licht durch den Lichtleiter, welches von einem fotoelektrischen Wandler empfangen wird.

Die Bewegungen des Lichtleiters während der Bewegung des Objektes oder des Menschen, an dem der Lichtleiter angebracht ist, erzeugen Änderungen in der Intensität des durch die Lichtleiter transmittierten Lichtes. Diese Intensitäts änderungen sind eine Funktion der Bewegung des Lichtleiters bzw. der optischen Faser und damit eine Funktion der Bewegungen, beispielsweise der respiratorischen Bewegung entsprechender Körperpartien eines Menschen.

Vorteilhafterweise wird zur Auswertung der detektierten Intensitätsänderungen ein Komparator verwendet, der die aktuellen Lichtamplituden mit einer vorher definierten Schwelle vergleicht und somit bestimmt, ob ein vorgegebener Wert überschritten wird oder nicht. Mittels dieses Komparators kann man die analoge Information der Bewegungsamplitude in die digitale Information Bewegung oder Ruhezustand (größer oder Kleiner als die Schwellenspannung), umwandeln.

Vorteilhafterweise kann ein weiterer Komparator vorgesehen sein, der eine Zeitspanne, die seit einem zuletzt detektierten Bewegungszustand verstrichen ist, mit einer vorbestimmten maximalen Zeitspanne vergleicht.

Als Alarm kann vorzugsweise ein optischer oder akustischer Alarm ausgegeben werden, z. B. wenn die seit der letzten Bewegungsänderung verstrichene Zeit einen vorher eingestellten maximalen Wert für einen gerade noch tolerablen vom Sollzustand abweichenden Istzustand überschreitet.

Für Die Durchleuchtung des Lichtleiters kann Licht in einem Wellenlängenbereich von 100 Nanometern bis 15 Mikrometern verwendet werden.

Der Alarm kann entweder unmittelbar bei einer vorzugsweise vorgesehenen Auswerteelektronik ausgelöst werden oder auch an einem von der Auswerteelektronik entfernten Ort, wenn sich z.B. Aufsichtspersonen in einem anderen Raum bef inden.

Die erfindungsgemäße Vorrichtung kann hierzu vorzugsweise eine kleine tragbahre mit einem Grundgerät in Kontakt stehende Alarmeinheit aufweisen, an die von der elektronischen Auswerteeinheit der Alarm übertragen wird. Dies kann durch Funk- oder Ultraschall oder (wenn eine entsprechende optische Verbindung möglich ist) auch durch Licht oder Infrarot übertragen werden. Je nach Anwendungsfall sollte hierbei sichergestellt sein, daß die tragbare Alarmeinheit das Alarmsignal kontinuierlich sicher empfangen kann. Es wird dazu in regelmäßigen, sinnvollen Zeitabständen eine Überprüfung der einwandfreien Übermittlungsfunktion erfolgen (Überprüfungsübermittlung, Testübermittlung).

Zusammenfassend ist insoweit festzuhalten, daß das erfindungsgemäße Verfahren und die erfindungsgemäße Vorrichtung den Effekt ausnutzen, daß Bewegungen einer optischen Faser zu einem Rauschen der transmittierten Energie in der Faser führen. Dementsprechend weist die erfindungsgemäße Vorrichtung eine elektronische Auswerteinheit auf, die diese Modulation des Lichtes aus dem Gesamtlicht extrahiert, aufbereitet und letztlich entscheidet, ob gerade ein vom Sollzustand abweichender nicht tolerabler Istzustand oder ein dem Sollzustand entsprechender Istzustand vorliegt.

Weitere Einzelheiten, Merkmale und Vorteile der Erfindung ergeben sich aus nachfolgender Beschreibung eines Ausführungsbeispiels anhand der Zeichnung.

Es zeigt:

Fig. 1A, 2A und 3A     eine erste Ausführungform einer erfindungsgemäßen Vorrichtung in Form einer Vorrichtung zum Überwachen der Atemtätigkeit eines Kindes;

Fig. 1B, g     eine zweite Ausführungsform der erfindungsgemäßen Vorrichtung, ähnlich derjenigen gemäß den Fig. 1A bis 3A;

Fig. 4     ein Blockdiagramm, das den Signalfluß und die einzelnen Schritte der Signalverarbeitung sowie die prinzipielle intrinsische Logik der Vorrichtung und des Verfahrens gemäß vorliegender Erfindung angibt;

Fig. 5     ein Signal am Ende eines Lichtleiters, wobei.die Lichtintensität über der Zeit zur Verdeutlichung der hohen Rauschsignalamplitude während der Bewegung aüfgetragen ist;

Fig. 6     ein Signal an einer Fotodiode bzw. am Ende eines Vorverstärkers (Stromspannungswandler), wobei die Spannung über der Zeit aufgetragen ist, um zu verdeutlichen, wie das Spannungsrauschen d.h. die Rauschsignalamplitude, während der Bewegung eine hohe Amplitude erreicht;

Fig. 7     ein Signal nach einem Hochpaß, wobei die Spannung über der Zeit aufgetragen ist und die Gleichspannungskomponente durch den Hochpaß entfernt wurde;

Fig. 8     ein Signal und eine Spannungsschwelle in einem Spannungskomparator, wobei die beiden zu vergleichenden Spannungen über der Zeit aufgetragen sind und wobei das Rauschen während der Bewegung die Schwelle übersteigt;

Fig. 9     Ein Ausgangssignal am Spannungskomparator, wobei die Ausgangsspannung über der Zeit aufgetragen ist und wobei der bistabile Komparator ein hohes Ausgangssignal (1 = wahr = hoch) ausgibt, wenn einem gewünschten Sollzustand entsprechende Istzustände vorhanden sind (z. B. Amplitude überschreitet Schwelle), und wobei ein niedriges Ausgangssignal (0 = unwahr = niedrig) ausgegeben wird, während der Phasen der unerwünschten Istzustände (z . B . Amplitude überschreitet Schwelle nicht);

Fig. 10     einen Vorschlag für eine statistische Anzeige, z.B. für die Zeitintervalle zwischen zwei Bewegungszuständen (Atemphasen, Phasen hoher Rauschamplitude); und

Fig. 11     ein Faserbündel mit einer alternativen Ausführungsform der Erfindung.

Das erfindungsgemäße Verfahren sowie die erfindungsgemäße Vorrichtung nutzen den Effekt, daß Licht, das durch Lichtleiter transmittiert wird, Intensitätsschwankungen (Transmissionsrauschen) bei Bewegungen des Lichtleiters aufweist. Die Ursache für das daraus resultierende Rauschsignal sind folgende:

1. Unterschiede in den Ausbreitungsbedingungen für Moden bei cokärentem Licht;
2. Unterschiede in der Geometrie der Totalreflektion innerhalb der Faser. In einer unbewegten Faser wird ein fester Anteil des Lichtes aufgrund von Totalreflexion durch die Faser transmittiert. Gleichzeitig erfüllt ein bestimmter Anteil des Lichtes die Bedingungen der Totalreflektion nicht an jeder Stelle, z.B. nicht an Biegungen, besonders wenn die Faser bis zum maximal möglichen Winkel mit Licht gefüllt ist. Der maximale Akzeptanzwinkel berechnet sich aus der numerischen Apertur gemäß einer bekannten (siehe z.B. EP-A-0436853, Seite 3, Zeilen 20 - 34) Formel.

$$\sin \alpha = NA = \sqrt{n_1{}^2 - n_2{}^2}$$

In dieser dem Fachmann bekannten Formel stellen $\alpha$ den maximalen Winkel zu der Längsachse der Faser, NA die numerische Apertur, $n_1$ und $n_2$ die Refraktionsindizes von Kern und Mantel der Faser dar.

Wenn die Faser bewegt wird, ändert sich die Geometrie der Faser. An Stellen, an denen zuvor Licht austrat, ist möglicherweise jetzt die Bedingung für Totalreflexion erfüllt. Umgekehrt ist an Stellen, an denen vorher Licht in der Faser blieb, die Bedingung für Totalreflexion aufgrund einer Biegung nicht mehr erfüllt und ein bestimmter Teil des Lichtes verläßt hier die Faser. Eine Änderung der Geometrie bedeutet also eine Änderung des Verhältnisses von transmittiertem zu verlorenem Licht in der Faser. Nur in einer völlig unbewegten Faser werden die Bedingungen für Totalreflexion an jedem Punkt der Faser die gleiche bleiben.

Die Transmission kann sich durch eine Änderung der Gesamt-Lichtlmenge ändern, sowie durch die Anzahl der Totalreflexionen in der Faser, wenn sich die Geometrie der Faser ändert. Beides ändert die Transmission des Lichtes.

Ein weiterer Mechanismus für Änderung in der Transmission einer Faser liegen in einer geringfügigen Deformierung der Faser an Biegungen. Dieser Effekt ist desto ausgeprägter, je weicher das Material ist, aus dem die Faser hergestellt wurde.

All diese Effekte, die zu Transmissionsänderungen in der bewegten Faser führen, so unerwünscht sie bei der Datenübertragung, insbesondere der analogen sind, werden beim erfindungsgemäßen Verfahren benutzt. Man kann den Effekt der Transmissions-Änderungen aufgrund von Änderungen in der Fasergeometrie durch geeignete Wahl des Lichtes, Wahl der Faser und der Einkoppelbedingungen optimieren. Dadurch wiederum kann der Effekt optimiert werden, mit dem gemäß den Prinzipien des erfindungsgemäßen Verfahrens Änderungen des Bewegungszustandes erfaßt und überwacht werden.

Im allgemeinen zeigt cohärentes Licht eine höhere Empfindlichkeit für Biegungen des, das Licht führende Lichtleiters als incohärentes Licht. Auch zeigen Lichtleiter mit kleinen numerischen Aperturen eine höhere EmpLichtleiter findlichkeit gegenüber Biegungen als Lichtleiter mit hohen numerischen Aperturen. Einzelfasern ergeben einen höheren Effekt als Faserbündel. Monomodefasern zeigen einen größeren Einfluß von Geometrieänderungen als Multimodefasern. Schließlich ist es wichtig, Licht unter allen Winkeln in die Faser einzuführen, bis hin zum maximalen Akzeptanzwinkel, d. h. die Faser maximal mit Licht zu füllen. Mit Monomodefasern kann man zusätzlich zu den oben beschriebenen allgemeinen Effekten noch daraus Nutzen ziehen, daß die Ausbreitung der Moden stark abhängig von der Geometrie der Faser ist. Die Verwendung von LED's ist günstig, weil sie Lambert-Strahler darstellen und damit die Faser unter allen erdenklichen Winkeln füllen und weil sie darüberhinaus sehr kostengünstig sind.

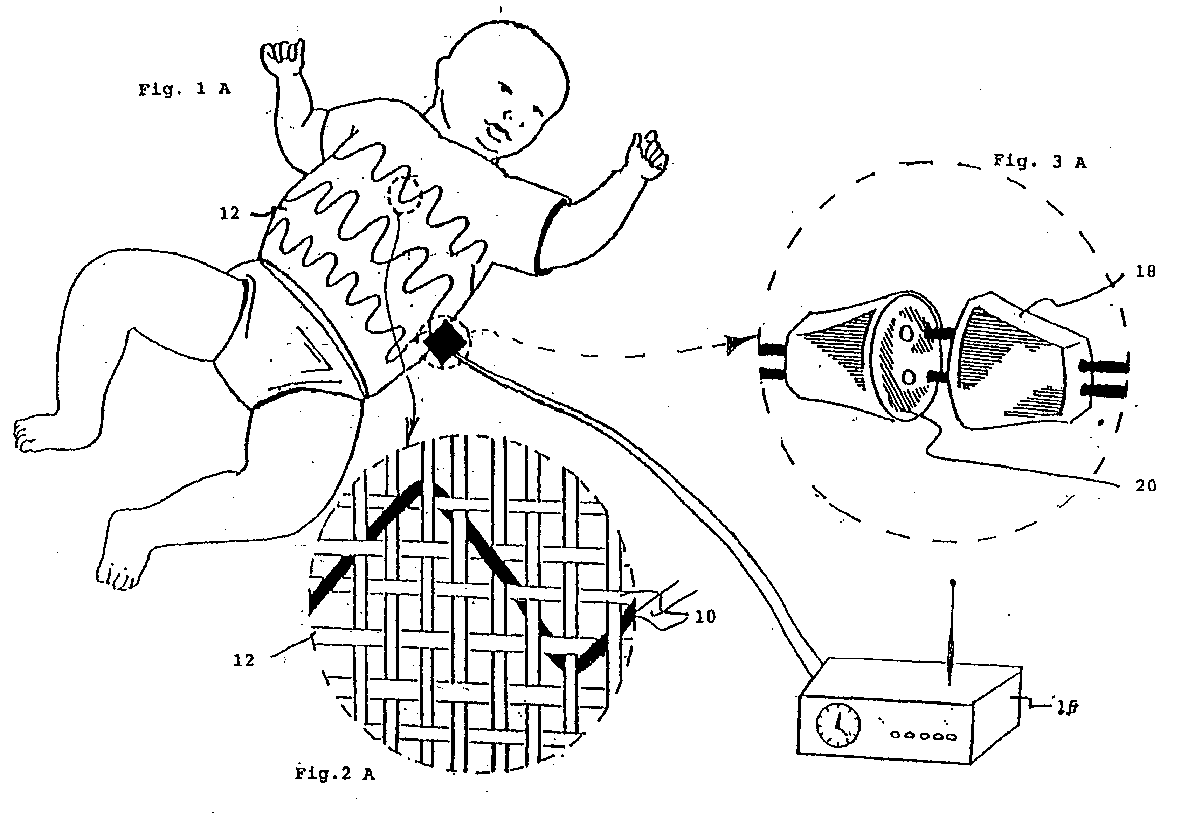

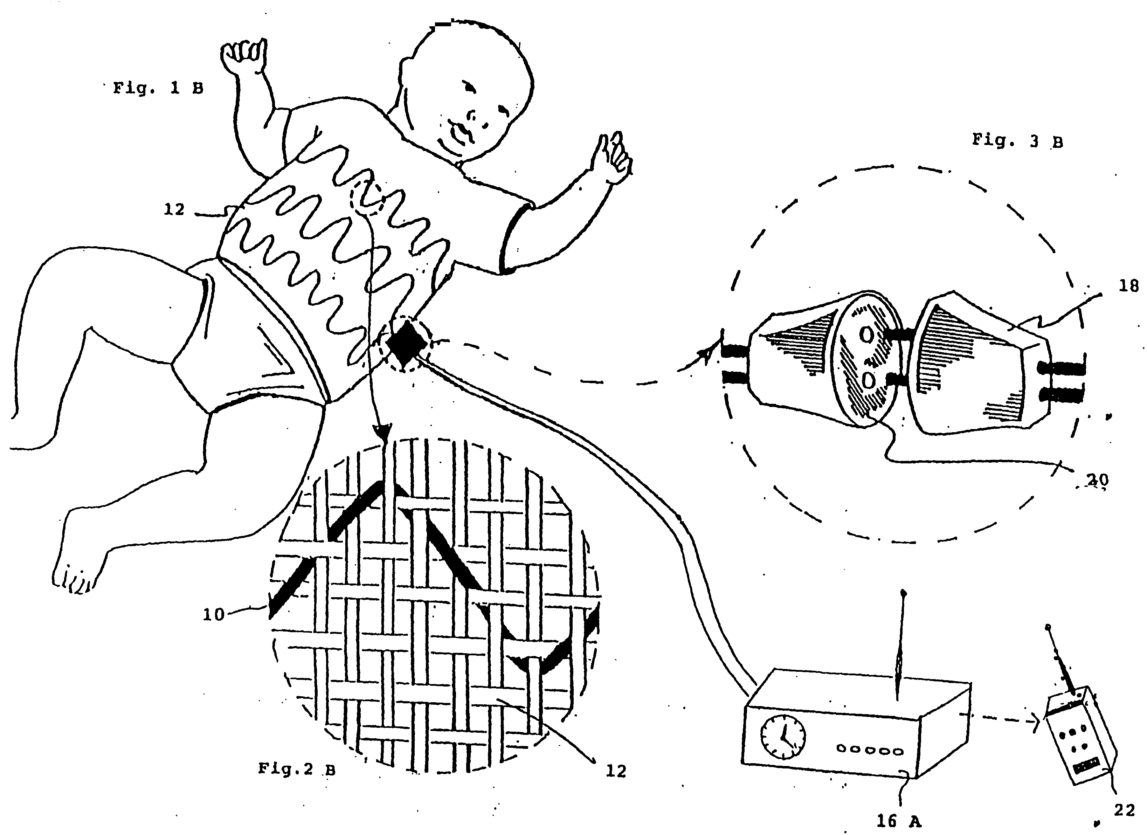

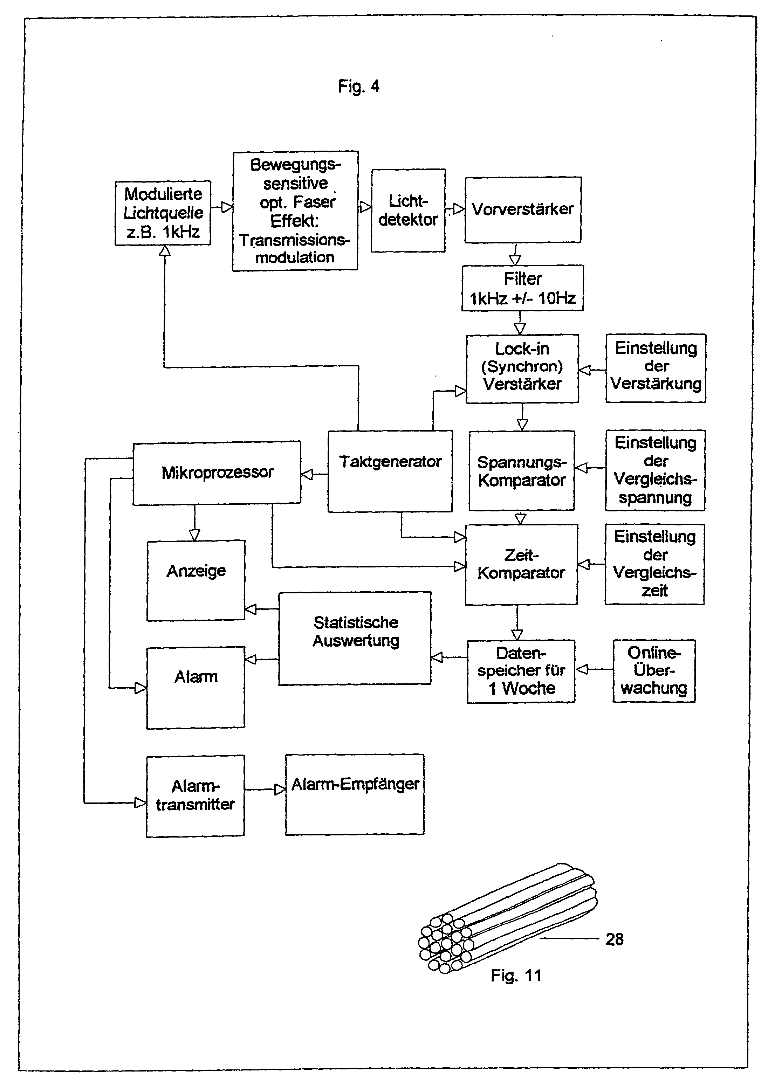

Entsprechend der in den Fig. 1A bis 3A ersten bevorzugten Ausführungsform der erfindungsgemäßen Vorrichtung wird eine einzelne optische Faser 10 im dargestellten Beispielsfalle (Vorrichtung zur Überwachung der Atembewegungen eines Kleinkindes) in innigen Kontakt mit dem kindlichen Brustkorb oder Bauch gebracht. Die Faser 10 ist aus einem Polymer wie z.B. PMMA (Polymethylmetacrylat) oder Glas oder Quarz in einem dünnen Kunststoffschlauch wie. z.B. TEFZEL (ein Warenzeichen der Fa. Dupont De Nemours of Wilmington Delware) angeordnet. Die optische Faser 10 hat einen Durchmesser zwischen 50 und 150 µm. Die Flexibilität der Faser nimmt mit abnehmenden Durchmesser zu und mit zunehmenden Durchmesser ab. Die Transmission und die Aufnahme des Lichtes steigen mit dem Quadrat des Faserdurchmessers. Die Faser wird bei der in den Figuren dargestellten bevorzugten Ausführungsform in ein eine Befestigungsvorrichtung bildendes dünnes Hemdchen 12 eingewebt oder auf andere Art und Weise befestigt. Vorzugsweise ist die Faser hierbei in einer sinusförmigen oder mäanderartigen Weise angeordnet, was die Länge der Faser in Kontakt mit den atembewegten Körperpartien des Kindes erhöht. Auf diese Weise übertragen sich die Bewegungen des Brustkorbes und/oder Bauches auf die Faser.

Wie oben beschrieben, erzeugen die Bewegungen der Faser eine einem Rauschen ähnliche Veränderung der Transmission des Lichtes in der Faser. Um dies zu nutzen, wird Licht einer geeigneten Lichtquelle (z.B. einer LED) in das eine Ende der Faser 10 eingekoppelt und wird am anderen Ende auf einen Fotodetektor gerichtet, z.B. eine Fotodiode. Dieser Fotodetektor empfängt das transmittierte Licht, verwandelt das optische in ein elektrisches Rauschsignal, das verstärkt und durch einen Hochpaß geschickt wird (Wechselstromkopplung). Es gilt ein geeignetes Frequenzband zu wählen, bevorzugt zwischen 0,1 und 10 Hertz, um die externen Einflüsse z.B. durch künstliches Licht, das beispielsweise aus Neonröhren kommen kann oder Einkupplungen der Netzfrequenz, so gering wie möglich zu halten, und um die im Vergleich zum Gesamtlicht kleinen Intensitätsänderungen detektieren zu können. Die Änderungen der Transmission im Frequenzband ereignen sich während der Atembewegungen. Wenn dieses Wechselsignal eine vorher bestimmte und/oder einstellbare Schwelle übersteigt, wird eine ausreichende Atmung unterstellt. Um noch mehr Unabhängigkeit vom Umgebungslicht zu bekommen, kann man die Lichtquelle mit einer Frequenz modulieren, die deutlich höher ist als die höchste im Umgebungslicht vorkommende Frequenz (z.B. doppelte Netzfrequenz, 120 Hz in den USA, 100 Hz in Europa), z.B. zwischen 1 und 10 kHz. In diesem Falle bedarf es einer Demodulationsstufe, um die besagte Trägerfrequenz zu extrahieren.

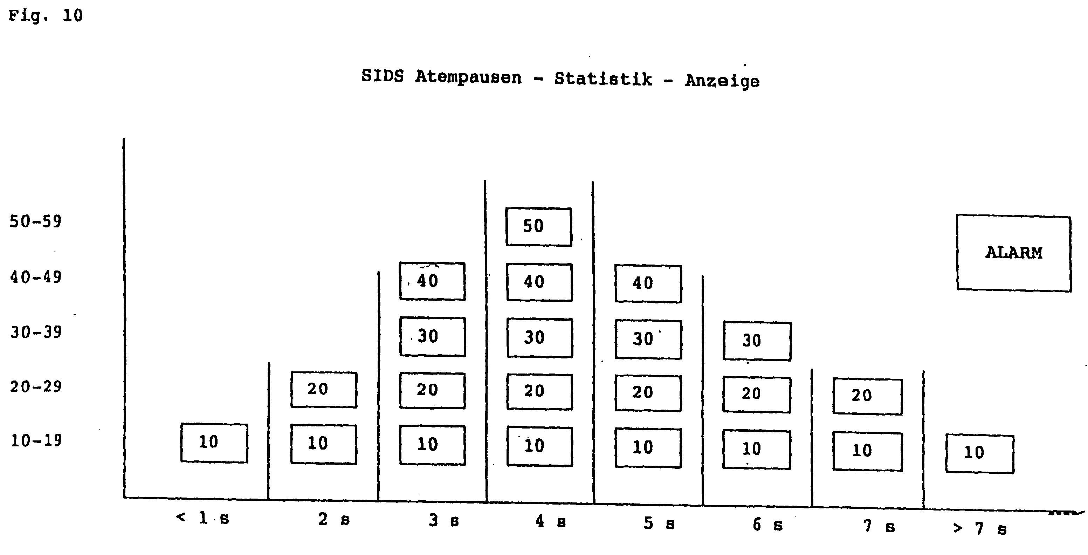

Eine elektronische Auswerteeinheit dirigiert auf diese Weise einen Alarm, wenn bei der in den Figuren dargestellten Ausführungsform die Atmung länger ausfällt als einem vorher eingestellten Fest-Wert entspricht. Dieses Zeitintervall kann entweder bei der Herstellung eingestellt werden oder von ärztlicher Seite oder eventuell durch die Eltern über eine entsprechende Einstellvorrichtung eingegeben werden. Wenn die Atmung für eine Zeit aussetzt, die länger als dieses eingestellte Intervall ist, wird der Alarm ausgelöst. Erfindungsgemäß wird der Alarm entweder als akustischer oder als optischer Alarm ausgegeben, entweder in unmittelbarer Nähe bzw. im auswertenden Gerät selbst 16A, oder in einer unabhängigen, vorzugsweise tragbaren Alarmeinheit 22, zu der hin der Alarm übertragen wird. Die Auswerteeinheit kann zusätzlich eine statistische Analyse über eine Bargraphenanzeige (SIDS-Alempausen-Statistik-Anzeige) ausgeben, die auch zum Teil verschiedene Zeitklassen und vertikal verschiedene Häufigkeitsklassen aufträgt (siehe Fig. 10).

Um das Überwachungsgerät so praktisch wie möglich einsetzen zu können, ist es vorteilhaft, wenn das Hemdchen 12 oder der Gürtel, die aus elastischem Material (Strechstoffe) bestehen und die Lichtleiter 10 enthalten, einfach von den verbindenden Lichtleitern getrennt werden kann, z.B. um das Hemdchen 12 zu waschen. Daher kann man den Lichtleiter 10 an der Stelle, an der er das Hemdchen 12 verläßt, mit Hilfe von möglichst leichten Plastiklichtleitersteckern 18, 20 trennen. Die optische Steckverbindung kann entweder für mechanischen Kontakt im Sinne einer End-zu-End-Verbindung 18, 20 sorgen oder es sind optische Elemente wie Linsen oder Stablinsen in die Stecker eingebaut, um die optische Verbindung herzustellen.

Eine andere Ausführung der Alarmeinheit des Gerätes beinhaltet eine tragbare Alarmeinheit 22 (sh. Fig. 1B bis 3B) und eine kleine tragbare batteriebetriebene Signaleinheit 16A, die z.B. an der elterlichen Kleidung angebracht wird oder an anderer Stelle, so daß ein von dem Gerät ausgegebener Alarm, der an die entkoppelte Alarmeinheit übertragen wird, von den Eltern oder von anderen für die Überwachung verantwortlichen Personen wahrgenommen werden kann. Um sicherzustellen, daß der Alarm jederzeit und überall übertragen werden kann, muß die Übertragung in regelmäßigen Abständen oder kontinuierlich überprüft werden. Dazu könnte das Gerät vorteilhafterweise z.B. einen kontinuierlichen, unmodulierten Träger aussenden, den die entkoppelte Alarmeinheit 22 kontinuierlich empfängt. Fällt der Empfang aus, gibt die Alarmeinheit 22 einen Alarm für eine defekte Kommunikation zwischen den Einheiten aus.

Eine andere Möglichkeit ist die regelmäßige Übertragung eines Überprüfungssignals (Testalarm). Die Übertragung zwischen den Geräten kann entweder per Funk, per Licht (inklusive Infrarotlicht) oder per Ultraschall erfolgen.

Eine bevorzugte Ausführungsform einer erfindungsgemäßen Vorrichtung, die insbesondere äußerst vorteilhaft als Überwachungsvorrichtung für die Atemtätigkeit eines Kindes ist, umfaßt folgende Komponenten (sh. Fig. 4).

A: Ein Netzteil oder eine (wiederaufladbare Batterie);

B: Eine Lichtquelle; d.h. eine Lichtaussendevorrichtung, die auch ultraviolettes oder infrarotes Licht aussenden kann; vorzugsweise wird eine LED mit 660 Nanometer Zentralwellenlänge verwendet, so daß das menschliche Auge das Licht kontrollieren kann;

C: Ein Lichtempfänger vorzugsweise in Form einer Fotodiode;

D: Ein Vorverstärker;

E: Einen Hoch- und einen Tiefpaßfilter, vorzugsweise zusammen als Bandpaßfilter;

F: Einen Verstärker;

G: Einen Spannungskomparator, der entscheidet, ob das Rauschen eine vorherbestimmte Schwelle überschreitet;

H: Eine Uhr, die einen Takt für ihren Mikroprozessor erzeugt und auch Zeitintervalle für den Vergleich mit der Zeit, die seit der letzten registrierten Atmung verstrichen ist, zur Verfügung stellt;

I: Einen Mikroprozessor;

J: Eine Alarmeinheit und/oder eine unabhängige, separate und entfernt aufgestellte weitere Alarmeinheit;

K:      Eine Anzeige zur Darstellung der statistischen Auswertung der Atemmuster.

L      Lichtleiter , Faser

Ferner kann diese besonderst bevorzugte erfindungsgemäße Vorrichtung einen Stecker für die Verbindung der optischen Fasern mit dem Lichtleiter im Hemdchen bzw. im Gürtel umfassen sowie ein wechselbares Hemdchen oder einen Gürtel oder eine sonstige geeignete Befestigungsvorrichtung, in die die optische Faser beispielsweise eingewebt ist.

Bei der alternativen in Fig. 11 dargestellten Ausführungsform ist die Einzelfaser durch ein Faserbündel 28 ersetzt, wobei die Fasern vorzugsweise durch einen elastischen, transparenten Klebstoff zu einem unlösbaren Bündel miteinander verbunden sein können. Dabei können sich die Elemente aus Fasern mit einem Einzeldurchmesser von 30 bis 50 $\mu m$ zusammensetzen und zwischen 30 und 1000 Einzellichtleiter enthalten.

Die erfindungsgemäße Vorrichtung kann eine Alarmeinrichtung umfassen, die einen Alarm immer dann aktiviert, wenn die Atmung des Kindes für eine Zeitdauer zum Stillstand kommt, die in vorbestimmtes, einstellbares Zeitintervall übersteigt. In diesem Falle fällt das Bewegungsrauschen der Faser unter eine voreingestellte Spannungsschwelle, was besagt, daß keine Atemexkursionen stattfinden. Der Alarm kann entweder optischer oder akustischer Art sein und entweder am Ort des Gerätes selbst oder entkoppelt in einer tragbaren Alarmeinheit ausgegeben werden. Die tragbare, entkoppelte Alarmeinheit empfängt die Information am besten in Form von Radiowellen (möglich sind auch Ultraschall oder Licht bzw. Infrarot) .

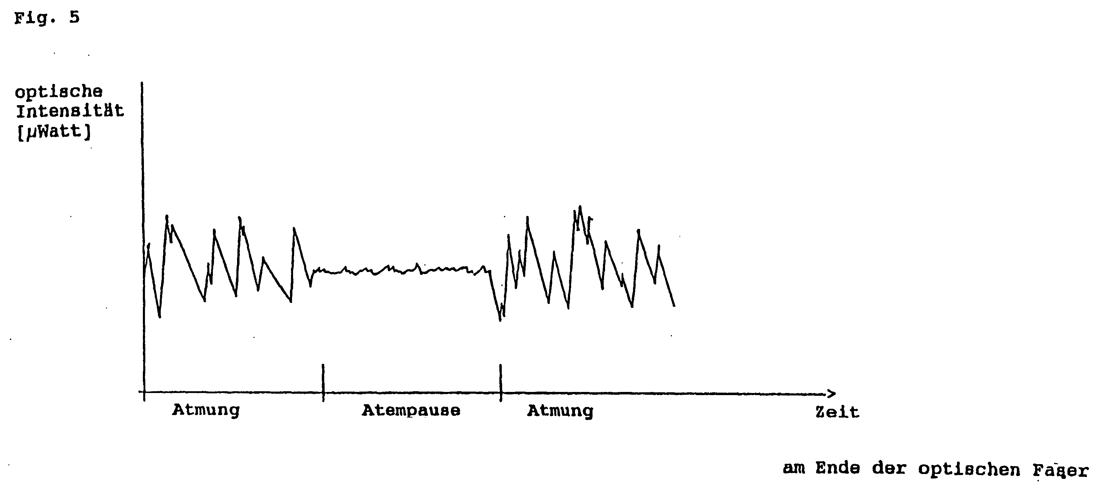

In den Fig. 5 bis 9 sind Signalverläufe dargestellt, die mittels einer Ausführungsform der erfindungsgemäßen Vorrichtung aufgenommen werden können, die als Überwachungsvorrichtung für die Atemtätigkeit eines Kindes in der zuvor beschriebenen Art und Weise ausgebildet ist.

Fig. 5 verdeutlicht hierbei die Lichtintensität, die gegenüber der Zeit aufgetragen ist. Fig. 5 verdeutlicht hierbei die hohe Rauschsignalamplitude während der Atmungsphasen.

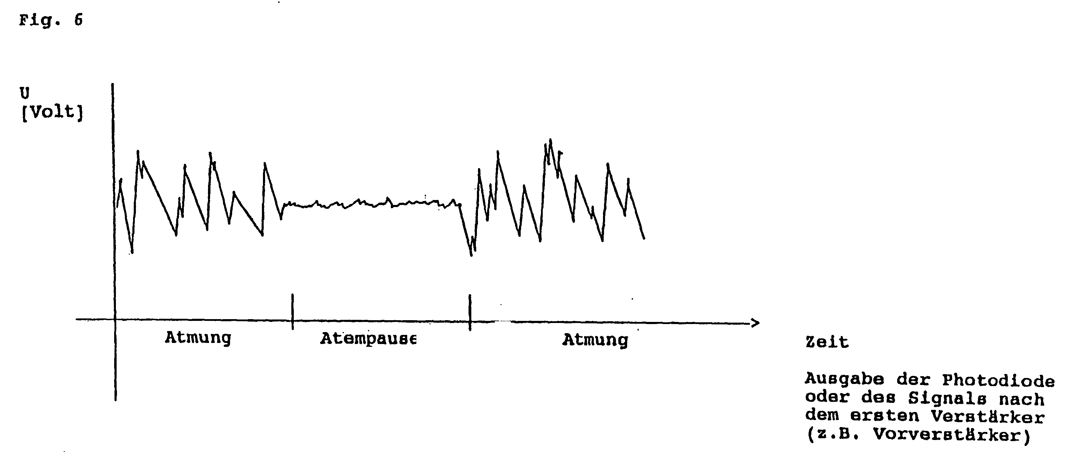

Fig. 6 zeigt das Signal an der Fotodiode bzw.. am Ende des Vorverstärkers. Hierbei ist ein Spannungssignal über der Zeit aufgetragen. Wiederum wird deutlich, wie das Spannungsrauschen während der Atembewegungen eine hohe Amplitude erreicht.

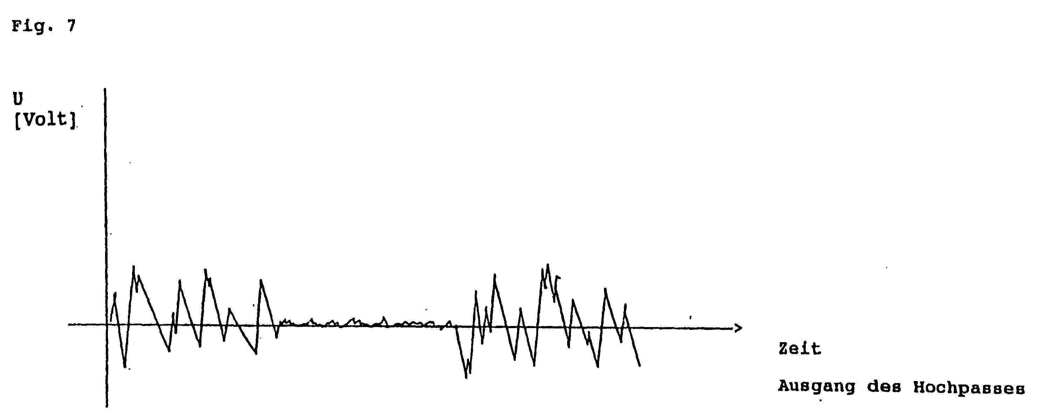

Fig. 7 zeigt das Signal nach dem Durchlauf durch einen Hochpaßfilter. Hierbei ist die Spannung über der Zeit aufgetragen, wobei die Gleichspannungskomponente durch den Hochpaßfilter entfernt wurde.

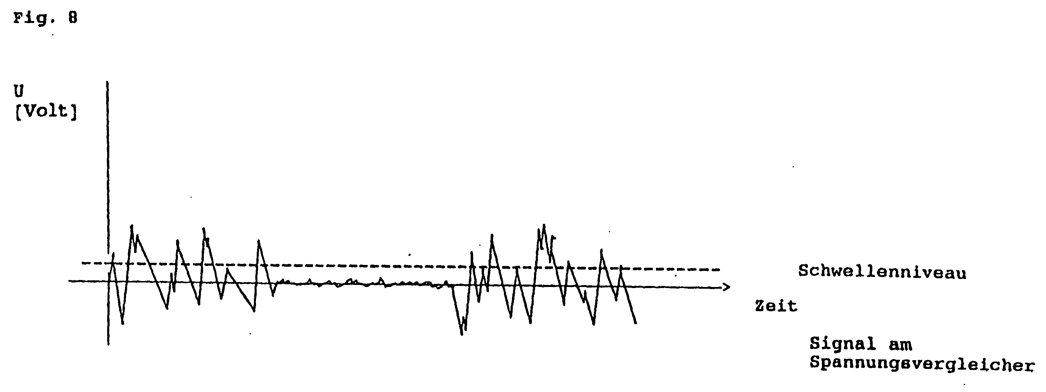

Fig. 8 zeigt das Signal und die Spannungsschwelle im Spannungskomperator. Es ist die Ausgangsspannung des Spannungskomperators über der Zeit aufgetragen, wobei das Rauschen während der Atembewegungen die Schwelle übersteigt.

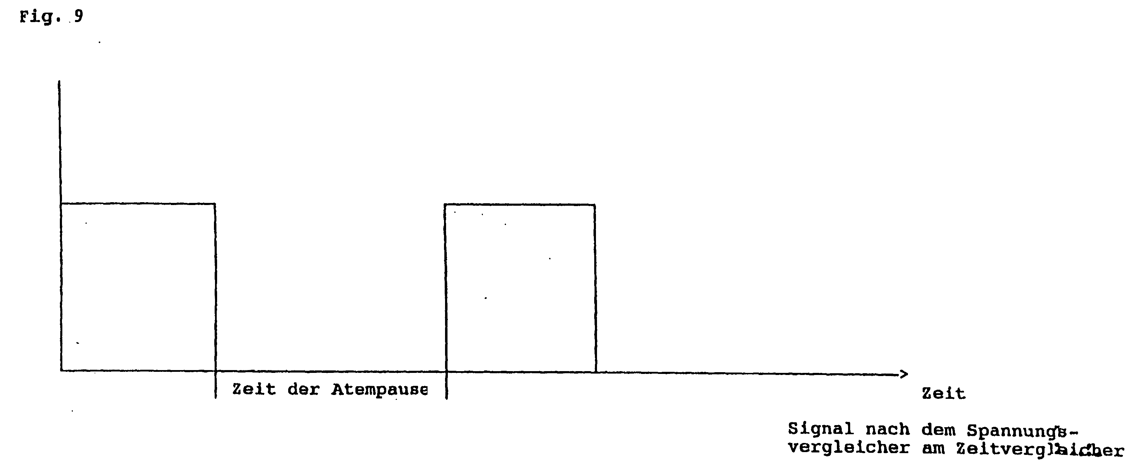

Fig. 9 zeigt das Ausgangssignal am Spannungskomperator. Die Ausgangsspannung ist über der Zeit aufgetragen, wobei der bistabile Komparator ein hohes Ausgangssignal ausgibt, wenn Atembewegungen vorhanden sind, und ein niedriges Ausgangsignal ausgibt, während der Atempausen oder umgekehrt.

Es ist bei Kleinkindern möglich, daß eine Disposition für den plötzlichen Kindstod existiert, so daß eine Dokumentation von Ereignissen insuffizienter Atmung interessant und wünschenswert sein kann. Deshalb kann die erfindungsgemäße Vorrichtung mit einem Halbleiterspeicher ausgerüstet sein und/oder mit einem zusteckbaren Drucker, mit dem man diese Vorkommnisse dokumentieren kann. Der Speicher könnte z.B. auf einer herausnehmbaren Speicherkarte untergebracht sein, die zur Auswertung in eine Klinik gebracht werden kann, so daß es nicht notwendig ist, das ganze Gerät zur transportieren. Während die Auswertung in der Klinik stattfindet, kann mit einer anderen Speicherkarte die Überwachung fortgesetzt werden.

Es gibt Klassen für die, Zeit, die zwischen den Atmungen verstreicht bzw. für die Zeit seit der letzten Atemexkursion, sowie Klassen bestimmter Häufigkeit. Eine bestimmte Zeitklasse und eine entsprechende Häufigkeitsklasse wird durch eine LED repräsentiert, die leuchtet, wenn die Klasse voll ist (Fig. 10). Wenn in einer Zeitklasse die maximale Auftretungswahrscheinlichkeit voll ist und die selbe Klasse nochmal vorkommt, müssen alle anderen Klassen in ihrer Häufigkeitsfrequenz reduziert werden. Dies führt zu einem kontinuierlichen Spektrum von Häufigkeiten und Zeitklassen, das eine statistische Auswertung des kindlichen Atemmusters darstellt.

Obwohl die erfindungsgemäße Vorrichtung und das erfindungsgemäße Verfahren voranstehend anhand einer Überwachungsvorrichtung für die Atemtätigkeit eines Kleinkindes beschrieben wurde, sind die gleichen erfindungsgemäßen Prinzipien auch auf anderen Gebieten, insbesondere im Gebiet der Technik zur Überwachung von Maschinen oder zur Erstellung von Bewegungsmeldern und Alarmanlagen, einsetzbar. Ggf. müssen Anpassungen bezüglich der Alarmanzeige vorgenommen werden, beispielsweise im Falle einer Alarmanlage zur Sicherung von Gegenständen, bei denen der Ruhezustand der gewünschte Sollzustand ist, während das Entfernen des Gegenstandes den zu detektierenden vom gewünschten Sollzustand abweichenden Istzustand darstellt. In einem solchen Falle wird dann der Alarm ausgelöst, wenn die Vorrichtung eine Bewegung detektiert, während sie im Falle der Überwachungsvorrichtung für Kleinkinder einen Alarm ausgibt, wenn keine Bewegung detektiert wird, wenn also Atemstillstand vorliegt. Eine weitere sehr vorteilhafte Anwendung ist im Sicherheits- bzw. Überwachungsbereich gegeben. Hier können z. B. sicherheitsrelevante Bewegungen von zu überwachenden Ausstellungsgegenständen, Maschenzäunen, Teppichböden detektiert werden, indem ein Lichthalter in engen mechanischen Kontakt mit dem zu überwachenden Gegenstand gebracht wird, z. B. an Bilderrahmen befestigt, entlang dem Maschendraht befestigt oder in Teppiche eingewebt wird, so daß jede Bewegung, also die Änderung aus dem Ruhezustand in einen kurzzeitigen Bewegungszustand detektiert werden kann in dem man den Lichtleiter durchleuchtet und durch Vergleich mit einer Schwellenspannung festlegt, ob eine Bewegung vorliegt oder nicht. Ein erfindungsgemäßer Vorzug dieser Überwachungseinrichtung liegt darin, daß z. B. sehr dünne Glasfasern nahezu unsichtbar verlegt werden können. Ein weiterer Vorteil der erfindungsgemäßen ausgebauten Sicherheitseinrichtung ist dadurch gegeben, daß auch auf die Zerstörung der Überwachungssensoren (Unterbrechung der Transmission) geprüft werden kann. Vorteilhaft ist auch, daß bei sehr langen Lichtleitern wie z. B. bei der Sicherung eines Maschenzaunes um einen Flughafen mittels TDR (Time Domain Reflection) Rückschlüsse auf den Ort der Störung (Tatort) geschlossen werden kann.

Wird der Lichtleiter in einem Teppichboden verlegt, befindet er sich sowohl ohne Einfluß eines Gewichtes (betreten des Teppichs) als auch mit konstanter Beeinflussung durch ein Gewicht (Schreibtisch, Schrank) im Ruhezustand. Jedes Betreten oder Gehen auf dem Teppich führt, wie oben beschrieben, zu einer Veränderung der Transmission und kann so eine erwünschte Funktion bewirken, z. B. einen Alarm auslösen oder einen Scheinwerfer einschalten.

**Patentansprüche**

1. Verfahren zur Überwachung der Änderung des Bewegungszustandes von Gegenständen oder Teilen des menschlichen Körpers, welches folgende Verfahrensschritte umfaßt:

   - Anordnen zumindest einer optischen Faser im gutem mechanischen Kontakt mit dem zu überwachenden Gegenstand oder Körperteil, so daß die Faser in Abhängigkeit von dem Bewegungszustand des zu überwachenden Gegenstandes oder Körperteiles sich bewegt;
   - Hindurchleiten des von einer Lichtquelle ausgesendeten Lichtes durch die optische Faser zu einem photoelektrischen Wandler;
   - Überwachen der Veränderungen der Intensität des transmittierten Lichtes, wobei die Intensität des transmittierten Lichtes als Funktion der Bewegung der Faser variiert;
   - Vergleichen der Intensitätsänderung des transmittierten Lichtes, die durch die Bewegung des zu überwachenden Gegenstandes oder Körperteiles hervorgerufen wird, mit einer vorbestimmten Schwelle, die das Entscheidungskriterium zwischen bewegtem und unbewegtem Zustand des zu überwachenden Gegenstandes oder menschlichen Körperteiles darstellt;
   - Auslösen eines Alarms, falls die vorbestimmte Schwelle überschritten wird,

   **dadurch gekennzeichnet, dass**

   - beim Hindurchleiten des von einer Lichtquelle ausgesendeten Lichtes durch die optische Faser zu einem photoelektrischen Wandler die Faser maximal mit Licht gefüllt wird, also das Licht unter allen Winkeln bis zum maximalen Akzeptanzwinkel der Faser in die Faser eingeleitet wird, wobei der maximale Akzeptanzwinkel so definiert ist, dass in einer unbewegten Faser Licht, das außerhalb dieses Winkels in die Faser eingeleitet wird, die Bedingungen für Totalreflexion nicht erfüllt und nicht durch die Faser transmittiert wird und
   - wobei die Lichtquelle mit einer Frequenz moduliert wird, die höher ist als 120 Hz und eine Demodulation durchgeführt wird,

2. Verfahren nach Anspruch 1, bei dem die optische Faser in einer Haltevorrichtung angeordnet wird, die auf dem zu überwachenden Gegenstand oder dem Körperteil befestigt wird.

3. Verfahren nach Anspruch 1 oder 2, bei dem die optische Faser in einem sinusförmigen oder mäanderartigen Verlauf angeordnet wird.

4. Verfahren nach einem der Ansprüche 1 bis 3, **dadurch gekennzeichnet, daß** das Licht, das durch die Faser transmittiert wird, eine Wellenlänge in einem Bereich von 0,1 bis 15 μm hat.

5. Verfahren nach einem der Ansprüche 1 bis 4, bei dem die Änderung der Lichttransmission mittels einer elektronischen Auswerteeinheit überwacht wird.

6. Verfahren nach Anspruch 5, bei dem der Alarm in der Nachbarschaft der elektronischen Auswerteeinheit ausgelöst wird.

**7.** Verfahren nach Anspruch 5, bei dem der Alarm entfernt von der Auswerteeinheit ausgelöst wird.

**8.** Verfahren nach einem der Ansprüche 1 bis 7, bei dem eine separate, entfernt angeordnete Alarmeinheit vorgesehen wird, welche einen Alarm auslöst, wenn ein Alarmsignal von der Auswerteeinheit zu der entfernt angeordneten Einheit übertragen wird.

**9.** Verfahren nach Anspruch 8, bei dem ferner ein Verifikationssignal zwischen der Auswerteeinheit und der separaten Alarmeinheit gesendet wird.

**10.** Verfahren nach einem der Ansprüche 1 bis 9, bei dem zur Atemüberwachung eines Menschen, insbesondere eines Kindes, eine Zeitspanne, die seit einer letzten Atembewegung verstrichen ist, mit einer vorgegebenen maximalen Atemstillstandszeit verglichen wird.

**11.** Verfahren nach Anspruch 10, bei der der Alarm ausgelöst wird, wenn die abgelaufene Zeitspanne eine vorbestimmte Atemstillstandszeit überschreitet.

**12.** Vorrichtung zum Überwachen der Änderung des Bewegungszustandes eines Gegenstandes oder menschlichen Körperteiles umfassend

- zumindest eine optische Faser (10), die im gutem mechanischen Kontakt mit dem Gegenstand oder dem Körperteil des zu überwachenden Menschen anzuordnen ist;
- eine Einrichtung zum Durchleuchten der optischen Faser, wobei die Faser maximal mit Licht gefüllt wird;
- eine Einrichtung zum Empfangen und Überwachen des durchleuchtenden Lichtes, wobei das empfangene Licht als Funktion der durch die Bewegungsänderung veränderten Anordnungsgeometrie der optischen Faser (10) variiert

**dadurch gekennzeichnet daß**

- beim Durchleuchten der optischen Faser die Faser maximal mit Licht gefüllt wird, also das Licht unter allen Winkeln bis zum maximalen Akzeptanzwinkel der Faser in die Faser eingeleitet wird, wobei der maximale Akzeptanzwinkel so definiert ist, dass in einer unbewegten Faser Licht, das außerhalb dieses Winkels in die Faser eingeleitet wird, die Bedingungen für Totalreflexion nicht erfüllt und nicht durch die Faser transmittiert wird und
- eine Modulationsstufe zum Modulieren der Lichtquelle mit einer Frequenz arbeitet, die höher ist als 120 Hz, sowie eine Demodulationsstufe aufweist.

**13.** Vorrichtung nach Anspruch 12, **gekennzeichnet durch** eine Befestigungsvorrichtung (12) für die optische Faser (10).

**14.** Vorrichtung nach Anspruch 12 oder 13, **gekennzeichnet durch** eine Alarmeinrichtung und eine Einrichtung zum Auslösen des Alarms als Funktion der überwachten Durchleuchtung.

**15.** Vorrichtung nach einem der Ansprüche 12 bis 14, **gekennzeichnet durch** eine elektronische Auswerteeinheit mit einem Sender und einer separaten, vorzugsweise tragbaren, entfernten Alarmeinheit und **durch** eine Einrichtung zum Iniziieren des Alarms gemäß einem Signal, das von der Auswerteeinheit ausgesendet wird.

**16.** Vorrichtung nach einem der Ansprüche 12 bis 15, **dadurch gekennzeichnet, daß** die optische Faser eine optische Monof ilamentfaser ist.

**17.** Vorrichtung nach einem der Ansprüche 12 bis 16, **dadurch gekennzeichnet, daß** die Faser (28) ein Bündel von optischen Faserfilamenten umfaßt.

**18.** Vorrichtung nach einem der Ansprüche 12 bis 17, **dadurch gekennzeichnet, daß** eine erwünschte Funktion, wie z. B. ein optischer und/oder akustischer Alarm ausgelöst wird.

**19.** Vorrichtung nach einem der Ansprüche 12 bis 19, **dadurch gekennzeichnet, daß** eine der Auswerteeinrichtung

## EP 0 573 765 B1

und der entfernt angeordneten Alarmeinrichtung zugeordnete Einrichtung zum Aussenden eines Verifikationssignals zwischen diesen vorgesehen ist.

**Claims**

1. A Method to monitor changes of the state of movement of objects or of parts of the human body, comprising the following steps of proceedings:

   • positioning of at least one optical fiber in close mechanical contact with the object or part of the body to be monitored so that the fiber moves according to the state of movement of the object or the part of the body to be monitored
   • transmitting light emitted by a light source through the optical fiber to a photoelectric converter
   • monitoring the changes in the intensity of the transmitted light, and by doing so the intensity of the transmitted light varies as a function of the movement of the fiber means;
   • comparing changes in the intensity of transmitted light which are caused by the movement of the object or the part of the body to be monitored, with a predetermined threshold, being the criterion to determine whether the object or the part of the human body to be monitored is in a state of motion or in a motionless state;
   • triggering an alarm, in case the predetermined threshold is crossed.

   thereby **characterized, that**

   • while transmitting light emitted by a light source through an optical fiber to a photoelectric converter, the fiber is filled with light up to a maximal manner, i.e. light is coupled into the fiber making use of all angles up to the maximum acceptance angle of the fiber, the maximum acceptance angle being defined so that in an motionless optical fiber light outside this maximum acceptance angle does not meet the conditions for total reflection and thus can not be transmitted through the fiber, and
   • whereby the light wave is modulated with a frequency being higher than 120 Hz and a demodulation is carried out

2. A method as set forth in claim 1, wherein the optical fiber is positioned in a support, which is fixed to the object or to the part of the body to be monitored.

3. A method as set forth in claim 1 and 2, wherein the optical fiber is fixed in a sinusoidal manner or in slight bends.

4. A method as set forth in claim 1 to 3, thereby **characterized that** the light transmitted trough the fiber means is in a wavelength range from 0.1 to 15 μm.

5. A method as set forth in claim 1 to 4, wherein the changes of light transmission are monitored by means of an evaluating electronic unit.

6. A method as set forth in claim 5, wherein the alarm is triggered adjacent to the evaluating electronic unit.

7. A method as set forth in claim 5, wherein the alarm is triggered remote from the evaluating electronic unit.

8. A method as set forth in claim 1 to 7, wherein a separate remote alarm unit is provided which triggers an alarm when an alarm signal is transmitted from the evaluating unit to the remote unit.

9. A method as set forth in claim 8, wherein further a verification signal is sent between the evaluating unit and the separate alarm unit.

10. A method as set forth in claim 1 to 9, wherein in order to monitor the respiration of a human being, especially of a child the time elapsed since the last respiratory effort is compared with a given maximum respiratory arrest time.

11. A method as set forth in claim 10, wherein an alarm is triggered, if the elapsed time exceeds a predetermined respiratory arrest time

12. An Apparatus for monitoring the state of motion of an object or a part of the human body, including

- at least one optical fiber (10) which is in well related mechanical contact with the object or the part of the body of the human to be monitored,

- an apparatus to transilluminate the optical fiber, whereby the fiber is filled with light up to a acceptance angle maximum,

- an apparatus for receiving and monitoring transilluminating light, wherein the received light varies as a function of changes in the geometry how the optical fiber means (10) is positioned caused by the state of motion,

thereby **characterized, that**

- while transmitting light through the optical fiber, the fiber is filled with light in a maximal manner, i.e. light is coupled into the fiber making use of all angles up to the maximum acceptance angle of the fiber, the maximum acceptance angle being defined so that in an motionless optical fiber light outside this maximum acceptance angle does not meet the conditions for total reflection and thus can not be transmitted through the fiber, and

- a modulation unit uses a frequency higher than 120 Hz to generate modulated light and, additionally has a unit for demodulation.

**13.** An apparatus as set forth in claim 12, **characterized by** a support (12) for the optical fiber (10).

**14.** An apparatus as set forth in claim 12 or 13, **characterized by** an alarm unit and a unit to trigger the alarm serving as a function of the monitored light transmission.

**15.** An apparatus as set forth in claim 12 to 14, **characterized by** an evaluating electronic unit with a transmitter and a separate, preferably portable, remote alarm unit and with means to initiate the alarm based on a signal which is transmitted by the evaluating unit.

**16.** An apparatus as set forth in claim 12 to 15, thereby **characterized that** the optical fiber means (28) is an optical monofilament.

**17.** An apparatus as set forth in claim 12 to 16, thereby **characterized that** the fiber means (28) is a bundle of fiber optic filaments.

**18.** An apparatus as set forth in claim 12 to 17, thereby **characterized that** a desired function, e.g. an optic and / or an acoustic alarm is triggered.

**19.** An apparatus as set forth in claim 12 up to 19, thereby **characterized that** a unit linked to the evaluating unit and the remotely positioned alarm unit is suited to emit a verification signal between these two units.


**Revendications**

**1.** Procédé destiné à contrôler la modification de l'état des mouvements d'objets ou de parties du corps humain, comprenant des opérations suivantes:

- disposer au moins une fibre optique ayant un bon contact mécanique avec l'objet ou la partie du corps à surveiller, afin d'assurer le mouvement de la fibre en fonction de l'état des mouvements de l'objet ou de la partie du corps à surveiller,

- conduire la lumière rayonnée à partir d'une source lumineuse à travers la fibre optique vers un transducteur photovoltaique ;

- surveiller les modifications d'intensité de la lumière transmise, cette intensité de la lumière transmise pouvant varier en fonction du mouvement de la fibre;

- comparer la modification de l'intensité de la lumière transmise provoquée par le mouvement de l'objet ou de la partie du corps à surveiller, ayant un seuil prédéfini qui représente le critère de décision entre l'état mouvementé ou non mouvementé de l'objet ou de la partie du corps humain à surveiller;

- déclencher une alarme en cas de dépassement du seuil prédéfini.

**caractérisé en ce que**

- lors du procédé de conduire la lumière émise par une source de lumière à travers de la fibre optique vers un transducteur photovoltaïque, la fibre est emplie de lumière au maximum, donc de la lumière est conduite dans la fibre sous tous les angles possibles jusqu'à l'angle d'admission de la fibre; l'angle d'admission maximal étant défini de sorte que, dans une fibre non mouvementée, la lumière qui est conduite dans la fibre en dehors de cet angle, ne remplisse pas les conditions de la réflexion totale et qu'elle ne soit pas transmise à travers la fibre, et

- l'onde de la lumière étant modulée d'une fréquence plus élevée que 120 Hz, et procédant à une démodulation.

2. Procédé selon la revendication 1, **caractérisé en ce que** la fibre optique est disposée dans un dispositif porteur fixé sur l'objet ou la partie du corps à surveiller.

3. Procédé selon la revendication 1 ou 2, **caractérisé en ce que** la fibre optique est disposée dans une contexture sinusoïdale ou méendrique.

4. Procédé selon l'une quelconque revendications 1 à 3, **caractérisé en ce que** la lumière, qui est transmise à travers la fibre, a une longueur d'ondes dans une plage de 0,1 jusqu'à 15 $\mu$m.

5. Procédé selon l'une quelconque revendications 1 à 4, **caractérisé en ce que** la modification de la transmission de lumière est surveillée à l'aide d'une unité d'évaluation électronique.

6. Procédé selon la revendication 5, **caractérisé en ce que** l'alarme est déclenchée au voisinage de l'unité d'évaluation électronique.

7. Procédé selon la revendication 5, **caractérisé en ce que** l'alarme est déclenchée d'une position éloignée de l'unité d'évaluation électronique.

8. Procédé selon l'une quelconque revendications 1 à 7, **caractérisé en ce qu'**il est prévu une unité d'alarme séparée, disposée à une position éloignée, qui déclenche une alarme lorsqu'un signal d'alarme est transmis de l'unité d'évaluation vers l'unité disposée à la position éloignée.

9. Procédé selon la revendication 8, **caractérisé en ce qu'**en plus de cela, un signal de vérification est transmis entre l'unité d'évaluation et l'unité d'alarme séparée.

10. Procédé selon l'une quelconque revendications 1 à 9, **caractérisé en ce que**, dans le but de contrôler la respiration d'une personne, particulièrement d'un enfant, une période passée depuis le dernier mouvement de respiration est confrontée à la durée maximale prédéfinie d'apnée.

11. Procédé selon la revendication 10, **caractérisé en ce que** l'alarme est déclenchée lorsque cette période périmée excède une durée prédéfinie d'apnée.

12. Dispositif destiné à surveiller la modification de l'état des mouvements d'un objet ou d'une partie du corps humain, comprenant

- au moins une fibre optique (10), étant disposée de sorte qu'elle ait un bon contact mécanique avec l'objet ou la partie du corps de la personne à surveiller;

- une installation destinée à mirer la fibre optique; la fibre étant emplie de lumière au maximum

- une installation destinée à recevoir et à surveiller la lumière mirée; la lumière reçue variant en fonction de la géométrie de disposition de la fibre optique (10), qui est modifiée par la modification des mouvements

**caractérisé en ce que**

- lors du procédé de mirer la fibre optique, celle-ci est emplie de lumière au maximum, donc de la lumière est conduite dans la fibre sous tous les angles possibles jusqu'à l'angle d'admission de la fibre; l'angle d'admission maximal étant défini de sorte que, dans une fibre non mouvementée, la lumière qui est conduite dans la fibre en dehors de cet angle, ne remplisse pas les conditions de la réflexion totale et qu'elle ne soit pas transmise à travers la fibre, et

- un niveau de modulation destiné à moduler la source de lumière travaille avec une fréquence plus élevée que 120 Hz, et qui dispose également d'un niveau de démodulation.

13. Dispositif selon revendication 12, **caractérisé en ce qu'**il y a un dispositif de fixation (12) pour la fibre optique (10).

14. Dispositif selon revendication 12 ou 13, **caractérisé par** une installation d'alarme et une installation qui déclenche l'alarme en fonction du procédé surveillé de mirer.

15. Dispositif selon quelconque revendications 12 à 14, **caractérisé par** une unité d'évaluation électronique qui est dotée d'un émetteur et d'une unité d'alarme séparée, de préférence portable, disposée à une position éloignée, ainsi que par une installation destinée à initier l'alarme suivant un signal émis par l'unité d'évaluation.

16. Dispositif selon quelconque revendications 12 à 15, **caractérisé en ce que** la fibre optique est une fibre optique monofilaire.

17. Dispositif selon quelconque revendications 12 à 16, **caractérisé en ce que** la fibre (28) comprend un faisceau de filaments fibreuses optiques.

18. Dispositif selon quelconque revendications 12 à 17, **caractérisé en ce qu'**une fonction souhaitée, comme p. ex. une alarme optique et/ou acoustique, est déclenchée.

19. Dispositif selon quelconque revendications 12 à 19, **caractérisé en ce qu'**il est prévu de disposer une installation assignée à l'installation d'évaluation et à l'installation d'alarme à position éloignée, ayant pour but d'émettre un signal de vérification entre celles-ci.

Fig. 1 A

Fig. 2 A

Fig. 3 A

Fig. 1 B

Fig. 3 B

Fig. 2 B

Fig. 4

Fig. 11

Fig. 5

optische
Intensität
[μWatt]

Atmung        Atempause      Atmung        Zeit

am Ende der optischen Faser

EP 0 573 765 B1

Fig. 6

U
[Volt]

Atmung                    Atempause                    Atmung

zeit

Ausgabe der Photodiode
oder des Signals nach
dem ersten Verstärker
(z.B. Vorverstärker)

Fig. 7

U
[Volt]

Zeit

Ausgang des Hochpasses

EP 0 573 765 B1

19

Fig. 8

U [Volt]

Schwellenniveau

Zeit

Signal am Spannungsvergleicher

EP 0 573 765 B1

Fig. 9

Zeit der Atempause

Zeit

Signal nach dem Spannungsvergleicher am Zeitvergleicher

EP 0 573 765 B1

Fig. 10

SIDS Atempausen - Statistik - Anzeige

EP 0 573 765 B1